(19) Europäisches Patentamt
European Patent Office
Office européen des brevets

(11) Veröffentlichungsnummer: **0 141 784**
**B1**

(12) **EUROPÄISCHE PATENTSCHRIFT**

(45) Veröffentlichungstag der Patentschrift: **07.10.87**

(51) Int. Cl.⁴: **C 02 F 3/34**

(21) Anmeldenummer: **84810524.3**

(22) Anmeldetag: **29.10.84**

(54) **Verfahren zum Abbau von s-Triazin-Derivaten in wässrigen Lösungen.**

(30) Priorität: **04.11.83 CH 5968/83**

(43) Veröffentlichungstag der Anmeldung: **15.05.85 Patentblatt 85/20**

(45) Bekanntmachung des Hinweises auf die Patenterteilung: **07.10.87 Patentblatt 87/41**

(84) Benannte Vertragsstaaten: **CH DE FR GB IT LI**

(56) Entgegenhaltungen:
**EP - A - 0 047 719**
**GB - A - 2 025 919**
**GB - A - 2 127 006**
**US - A - 3 926 795**

**JOURNAL OF AGRICULTURAL AND FOOD CHEMISTRY, Band 32, Nr. 3, Mai/Juni 1984, Seiten 581-585, American Chemical Society, Washington, US; A.M. COOK u.a.: "Deethylsimazine: Bacterial dechlorination, deamination and complete degradation"**
**CHEMICAL ABSTRACTS, Band 96, Nr. 20, 17. Mai 1982, Seite 348, Nr. 167981q, Columbus, Ohio, US; A.M. COOK et al.: "Degradation of s-triazines: a critical view of biodegradation"**

(73) Patentinhaber: **CIBA-GEIGY AG, Klybeckstrasse 141, CH-4002 Basel (CH)**

(72) Erfinder: **Cook, Alasdair MacLeod, Dr., Etzelstrasse 61, CH-8820 Wädenswil (CH)**
Erfinder: **Hütter, Ralf, Prof. Dr., Rüttistrasse 72, CH-8044 Gockhausen (CH)**

**EP 0 141 784 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft einen neuen Mikroorganismus des Genus Rhodococcus, seinen Einsatz in einem Verfahren zum mikrobiologischen Abbau von Amino-s-Triazin-Verbindungen und von Cyanursäure in Abwässern sowie ferner ein Verfahren zum mikrobiologischen Abbau von s-Triazin-Derivaten unter kombinierter Verwendung dieses Mikroorganismus und von Pseudomonas spp. zu Zellmasse bzw. zu Zerfallsprodukten wie $NH_4^+$ und/oder Chlorid.

Bei der grosstechnischen Produktion von 1,3,5-Triazin-Verbindungen (= s-Triazin-Verbindungen) in der chemischen Industrie fallen laufend wässrige Lösungen an, die u.a. Chlor-s-triazin-, Amino-s-triazin- und (Cyclo) Alkylamino-s-triazin-Verbindungen als Verunreinigungen enthalten. Derartige Abwässer stellen aufgrund der in der Natur nur langsam abbaubaren s-Triazin-Derivate eine Belastung für die Umwelt dar. Eine Vorreinigung stellt aber eine mit einem extrem hohen Aufwand an Kosten und Energie verbundene Aufgabe dar, die bisher nicht zufriedenstellend gelöst werden konnte. In konzentrierten Abwässern anfallende s-Triazin-Derivate lassen sich durch Hydrolyse bei hoher Temperatur teilweise umwandeln. Im allgemeinen aber liegen die Rest-s-Triazin-Derivate aus der Produktion in sehr verdünnten Lösungen vor, so dass sie durch chemische Massnahmen kaum noch erfassbar sind.

Auf der Suche nach Reinigungsmethoden bei verdünnten Abwässern dieser Art ist man daher ständig bestrebt, mikrobiologische Verfahren zu entwickeln, die beim stufenweisen Abbau der s-Triazin-Derivate eine hohe Abbauleistung in jeder dieser Stufen erzielen. Nur von solchen Massnahmen und Reaktionsschritten soll im folgenden die Rede sein. Untergeordnete Nebenreaktionen mit sehr viel geringeren Reaktionsgeschwindigkeiten werden nicht speziell erwähnt.

Der erste Teil vorliegender Erfindung betrifft verschiedene Aspekte eines mikrobiologischen Teilausbaus von Amino-s-triazinen der Formel Ia, worin $R_1$ ein Chloratom oder eine $NH_2$-Gruppe und $R_2$ Wasserstoff oder einen aliphatischen oder cycloaliphatischen Kohlenwasserstoffrest mit maximal 4 Kohlenstoffatomen bedeuten, zu entsprechenden Ammelinen der Formel Ib und zu Ammeliden der Formel Ic.

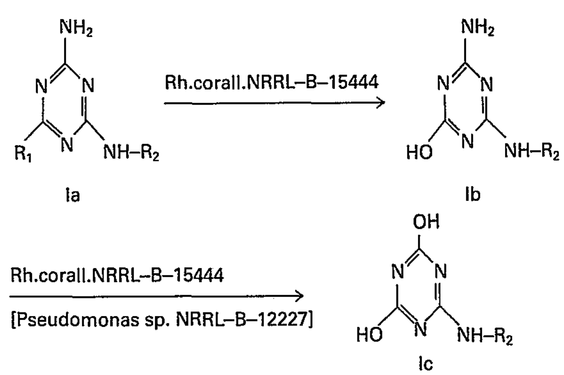

Ia Ib Ic

Ein Gegenstand vorliegender Erfindung ist der neue Mikroorganismus Rhodococcus corallinus Stamm NRRL B–15444, der aus Bodenproben von schweizerischen Agrar-Versuchsstationen der CIBA-GEIGY AG stammt, bei denen Böden mit unterschiedlicher Bepflanzung (Äpfel, Reben, Mais) über mehrere Jahre (4–19 Jahre) mit Simazin (= 2-Chlor-4,6-bis-äthylamino-s-triazin) und/oder mit Atrazin (= 2-Chlor-4-äthylamino-6-isopropylamino-s-triazin) behandelt worden waren. Dieser Bakterienstamm wurde am 13. Juni 1983 bei der Agricultural Research Culture Collection (NRRL) in Peoria, Illinois 61604, USA, deponiert. Rh.corallinus NRRL B-15444 vermag 2,4-Diamino-s-triazin- und 2-Amino-4-chlor-s-triazin-Derivate der Formel Ia zu desaminieren bzw. zu dechlorieren und damit in entsprechende Ammelin-Derivate der Formel Ib und in teilweise parallel verlaufender, hauptsächlich aber in nachfolgender Reaktion in Ammelid-Derivate der Formel Ic umzuwandeln.

Sofern $R_2$ Wasserstoff bedeutet, baut Rh. corallinus NRRL–B–15444 das Amelid I weiter über die Stufe der Cyanursäure zu Zellmasse ab. Die Erfindung erstreckt sich auch auf die Mutanten des Rh. corallinus NRRL–B–15444, die zu den gleichen Abbau-Reaktionen befähigt sind.

Es sind bereits Pseudomonas spp. und Sporothrix spp. bekannt geworden, die, wie im obigen Schema angedeutet, Amino-Substituenten in s-Triazinen in Hydroxylgruppen verwandeln können, die also Amino-s-Triazine in entsprechende Ammeline und Ammelide abzubauen vermögen. Mit der Auffindung des neuen Mikroorganismus Rh. corallinus NRRl B–15444 ist aber überraschenderweise auch der Zugang zum mikrobiologischen Abbau von Chlor-s-Triazinen eröffnet und damit erstmalig* die biologische Eliminierung des Chloratoms und die Bildung entsprechender Ammeline der Formel Ib möglich geworden.

* (Eine diesbezügliche Arbeit von Couch et al. [Proc. South Weed Sc. Soc. 1965, Vol. 18, p. 623] über eine mögliche Dechlorierung von Atrazin mit Hilfe eines Pilzes gibt nur ungenügende Hinweise zur Reproduzierung des Versuchs und hat in der Praxis keine Bedeutung erlangt.)

Rh. corallinus NRRL B–15444 besitzt gleichzeitig die vorteilhafte Eigenschaft, Cyanursäure zu spalten, ein Produkt, das in der Abbaukette von s-Triazinen eines der letzten Umwandlungsprodukte darstellt, und diese über Zerfallsprodukte wie Harnstoffe, Biuret, $NH_4^+$, in Zellmasse umzuwandeln.

Ein weiterer Gegenstand vorliegender Erfindung ist demgemäss das Verfahren zur mikrobiologischen Umwandlung von 2-Amino-s-triazin-Derivaten der Formel Ia in entsprechende Ammeline der Formel Ib und Ammelide der Formel Ic mit Hilfe des Mikroorganismus Rh. corallinus NRRL B–15444 und seiner entsprechenden Mutanten unter aeroben Bedingungen.

Ein weiterer Gegenstand vorliegender Erfindung ist auch ein Verfahren zum vollständigen mikrobiologischen Abbau von Cyanursäure mit Hilfe von Rh. corallinus NRRL B–15444 und seinen entsprechenden Mutanten unter aeroben Bedingungen.

In den vorstehenden Formeln Ia/IB sind unter dem Substituenten $R_2$ insbesondere Wasserstoff, Ethyl, Isopropyl und Cyclopropyl zu verstehen.

Unter den für die Praxis sehr wichtigen Produkten der Formel Ia ist insbesondere das 2-Amino-4-ethylamino-6-chlor-s-triazin zu nennen, das biologisch sehr leicht durch Desethylierung von 2,4-bis-ethylamino-6-chlor-s-triazin (= Simazin) und durch Des-isopropylierung von 2-Chlor-4-ethylamino-6-isopropylamino-s-triazin (= Atrazin) gebildet wird (Wichman und Byrnes, Weed Sc. 1975, Vol. 23, 488–453) und in Rinnsalen und Wasserläufen nachgewiesen wird, die aus mit Chlor-s-Triazin-Herbiziden behandelten Feldern stammen (Muir und Baker, J. Agric. Food Chem. 1976, Vol. 24, 122–125).

Der erfindungsgemäss zu verwendende Mikroorganismus wird zunächst auf einem geeigneten Basismedium gezüchtet und anschliessend wässrigen Lösungen der abzubauenden Substrate zugesetzt oder er wird direkt auf Medien, welche die abzubauenden Substrate enthalten, gezüchtet.

Das Wachstum des Rh. corallinus NRRL–B–15444 wird durch die Gegenwart gewisser Mengen Salze wie Sulfate oder Phosphate von Alkali–(K, Na), Erdalkali-(Ca, Mg) oder Übergangsmetallen (Fe) sowie Alkalimolybdate begünstigt.

Ein geeignetes Basismedium ist beispielsweise wie folgt zusammengesetzt:

| | |
|---|---|
| Kaliumphosphat-Puffer, pH 7,3 | 10 mM |
| Magnesiumsulfat-Heptahydrat | 0,25 mM |
| Spurenelementlösung nach Pfennig und Lippert (Arch. Microbiol. 55, 246, 1966), ergänzt mit 100 mg pro Liter Calciumchlorid-Dihydrat | 5 ml/Liter |
| Glycerin | 10 mM |
| Stickstoffquelle | 2,5 mM Stickstoff |

Als Kohlenstoffquelle kommen beispielsweise Glycerin, Fruktose, D-Gluconat, Azetat, Fumarat, Benzoesäure, Salicylsäure, m-Hydroxybenzoesäure u. a. in Frage (s. Tabelle 2.2 unten).

Als Stickstoffquellen werden die s-Triazin-Verbindungen der Formeln Ia bzw. Ib verwertet, darunter beispielsweise 2-Amino-4-ethylamino-6-chlor-s-triazin, Melamin, 2,4-Diamino-6-cyclopropylamino-s-triazin oder 2,4-Diamino-6-hydroxi-s-triazin.

Isolierung und Anreicherung

Der Ur-Typus der erfindungsgemäss verwendeten Mikroorganismen wird aus Abwasser- und Bodenproben gewonnen und von dorther durch Weiterzüchtung selektioniert.

1. Isolierung

1.1 Die Abwasserproben wurden 10 Min. lang bei +4 °C zentrifugiert (20000 g). Die überstehende Flüssigkeit wurde verworfen und der verbleibende Bodensatz (Pellet) in Pufferlösung (10 mM Kaliumphosphat-Puffer enthaltend 0,25 mM Magnesiumsulfat, pH 7,3) resuspendiert, abfiltriert und zusätzlich 2 mal mit Puffer gewaschen. Das Pellet wurde dann in einer gepufferten Salzlösung, beispielsweise in dem oben beschriebenen Basismedium, suspendiert und als Inoculat verwendet.

1.2 Bodenproben von 5 g aus verschiedenen Versuchsstationen der CIBA-GEIGY AG in der Schweiz, welche mehrere Jahre hindurch mit s-Triazinherbiziden behandelt worden waren, wurden in 100 ml der unter 1.1 beschriebenen Pufferlösung 1 Stunde bei 30 °C geschüttelt und dann 30 Min. absetzen gelassen. Anschliessend wurde die überstehende Flüssigkeit durch ein Filter (Whatman No. 1) gefiltert und das Filtrat wie unter 1.1 beschrieben zur Verwendung als Inoculat weiterbearbeitet.

2. Anreicherung

Proben von 3 ml Lösung, enthaltend 2,5 mM Stickstoff (in Form von s-Triazinverbindungen), 5 mM Glukose, 5 mM Succinat, 10 mM Glycerin und 0,4 ml Inoculat-Lösung, werden in ein Kulturröhrchen eingeschlossen und in absolut Stickstoff-freier Umgebung in einem Behälter, gefüllt mit 20% (V/V) Sauerstoff und 80% (V/V) Helium, unter einem Druck von ca. $8 \times 10^4$ Pa bei 30 °C 1 bis 3 Tage inkubiert. Daraus anschliessend entnommene Proben wurden auf Agarplatten übertragen und nach positiver Prüfung selektiert und isoliert.

Charakterisierung des neuen Mikroorganismus Rhodococcus corallinus NRRL B–15444

1. Allgemeine Parameter und Mikroskopie

Rhodococcus corallinus ist grampositiv, Oxidase-negativ und wächst vorzugsweise bei 20° bis 45 °C im bevorzugten pH-Bereich von 6–8. Er bildet tiefrote Kolonien, einzelne Mutanten treten auch in helleren Farbtönen auf. Sein Enzymsystem vermag in s-Triazin-Derivaten der Formel Ia das Cl-Atom gegen eine Hydroxylgruppe auszutauschen.

Als Stickstoff-Quellen kann er die freien Aminogruppen der s-Triazin-Derivate der Formel Ia und Ib, darunter Melamin, Ammelin, Ammelid, 2,4-Diamino-6-chlor-s-triazin, Cyanursäure, Biuret, Harnstoff, $NH_4^{\oplus}$ verwerten und als Kohlenstoff-Quelle beispielsweise Glycerin, Essigsäure, Ethanol u. a.

In der lichtmikroskopischen Abbildung ist Rhodococcus corallinus als ein nichtmotiles Stäbchen von 1–2 µm zu erkennen, das nach der Zellteilung häufig in V- oder Y-Formen vorkommt. Im elektronen-mikroskopischen Bild erscheint der Mikroorganismus in der V-Form, die entsprechend dem grampositiven Merkmal die flache Oberfläche sowie ausserdem den Zellteilungsring aufweist.

2. Biochemische Charakterisierung und Klassifizierung des neuen Mikroorganismus

Biochemische Teste

2.1 Zur allgemeinen biochemischen Charakterisierung und Klassifizierung von Rhodococcus corallinus NRRL B–15444, werden zwei käufliche Systeme verwendet. Sie liefern für grampositive Organismen allgemeine Charakteristik-Informationen.

«Oxi/Ferm Tube» und «Enterotube-II» (Roche-Diagnostik)

| Eigenschaften: | + + beide Teste positiv<br>– – beide Teste negativ<br>+ – ein Test positiv, ein Test negativ |
|---|---|
| Anaerober Dextrose-Abbau | – – |
| Gasbildung bei anaerobem Dextrose-Abbau | – – |
| Lysindecarboxylase | – – |
| Ornithindecarboxylase | – – |
| $H_2S$-Bildung | – – |
| Indol-Bildung | – – |
| Adonit-Abbau | – – |
| Lactose-Abbau | – – |
| Arabinose-Abbau | – – |
| Sorbit-Abbau | – – |
| Acetoin-Bildung | – – |
| Dulcit-Abbau | – – |
| Phenylalanindesaminase | – – |
| Urease | + + |
| Simmons-Citratverwertung | – – |
| Argininhydrolase | – – |
| $N_2$-Produktion | – – |
| Xylose-Abbau | – – |
| Aerober Dextrose-Abbau | – – |

2.2 Charakterisierung (nach H. Seiler J. General Microbiol. (1983) 129, 1433–1471

| Eigenschaften | |
|---|---|
| Azetat als C-Quelle<br>Propionat als C-Quelle | + |
| Valerat als C-Quelle<br>Capronat als C-Quelle | + |
| Adipinat als C-Quelle<br>Levulinat als C-Quelle | + |
| Glyoxylat als C-Quelle<br>Glycin als C-Quelle<br>Xanthin-Hydrolyse | – |
| D-Xylose als C-Quelle<br>D-Galaktose als C-Quelle | – |
| Säurebildung aus D-Glukose<br>Säurebildung aus Sukrose | – |
| Säurebildung aus Laktose<br>Säurebildung aus Stärke<br>Säurebildung aus Dextrin | – |
| Anaerobische Reaktion | – |
| 5-Aminovalerat als C-Quelle | – |
| Succinat als C-Quelle | + |
| Adipinat als C-Quelle | – |
| Citrat als C-Quelle | + |
| D-Glukonat als C-Quelle | + |
| L-Leucin als C-Quelle | – |
| L-Asparagin als C-Quelle | – |
| L-Aspartat als C-Quelle | – |
| D-Ribose als C-Quelle | – |
| L-Arabinose als C-Quelle | – |

Konservierung von Rhodococcus corallinus NRRL B–15444

Für die Konservierung des neuen Stammes eignen sich folgende Methoden:

a) Aufbewahrung des Zellmaterials des Stammes auf Schräg-Agar (gemäss dem weiter oben beschriebenen Basismedium; N-Quelle des 2-Cl-Triazins Ia obligatorisch) und

b) Lyo-Ampullen (Neben der C-Quelle ist die Anwesenheit geringer Mengen 2-Cl-Triazin als gewünschte N-Quelle obligatorisch). Die Kultur wird von der Nährlösung abzentrifugiert und die Zellmasse in 1/4 bis 1/3-Volumen 15%iger Magermilch resuspendiert und lyophilisiert.

Von dem neuen Stamm können sich spontan Mutanten bilden oder es lassen sich künstlich Mutanten herstellen, die ebenso wie der natürliche Stamm Verbindungen der Formel Ia bzw. Ib in wässriger Lösung abzubauen vermögen und Zellmasse produzieren. Mutanten kann man auch chemisch, beispielsweise mit bestimmten Guanidinderivaten, z.B. N-Methyl-N'-nitro-N-nitrosoguanidin oder Alkalinitrit, z.B. Natriumnitrit, oder physikalisch, z.B. durch Ultraviolett-, Röntgen- oder radioaktive Strahlung, erzeugen.

Die vorliegende Erfindung betrifft im zweiten Teil verschiedene Aspekte eines mikrobiologischen Totalabbaus von Amino-s-triazinen der Formel Ia und Ib, worin $R_1$ ein Chloratom oder eine $NH_2$-Gruppe und $R_2$ Wasserstoff oder einen aliphatischen oder cycloaliphatischen Kohlenwasserstoffrest mit maximal 4 Kohlenstoffatomen bedeuten, zu hauptsächlich Zellmasse, durch kombinierte Anwendung der Mikroorganismen

a) – Rhodococcus corallinus NRRL–B–15444 mit wahlweise

b) – Pseudomonas sp. NRRL–B–12228 und/oder

c) – Pseudomonas sp. NRRL–B–12229, unter gegebenenfalls weiterem Zusatz von

d) – Pseudomonas NRRL–B–11308 und/oder

e) – Sporothrix schenkii NRRL–Y–11307.

Die unter b) und c) genannten Pseudomonas spp. und ihre Abbau-Daten sind aus der EP-A 47719 (oder aus der ihr äquivalenten ZA-A. 81/6236) bekannt geworden.

Die unter d) und e) genannten Mikroorganismen und ihre Abbau-Daten sind aus der DE-A 2923794 (oder aus der ihr äquivalenten GB-A 2,025,919) bekannt geworden.

Wie im folgenden beschrieben, erzielen Mischkulturen aus vorstehend genannten Mikroorganismen höhere Abbauleistungen, als von der Addition der verschiedenen Einzel-Abbauleistungen der Mikroorganismen her zu erwarten gewesen wäre.

Die Erfindung betrifft also auch ein kombiniertes mikrobiologisches Verfahren, durch welches in wässrigen Lösungen, z.B. Abwässern, enthaltene s-Triazin-Derivate der Formel Ia oder Ib unter aeroben Bedingungen durch die gemeinsame Behandlung dieser Lösungen mit Rhodococcus corallinus NRRL–B–15444 oder seinen Mutanten zusammen mit einem der Pseudomonas spp. NRRL B–12228 oder NRRL B–12229 oder ihren Mutanten unter gegebenenfalls weiterem Zusatz von Pseudomonas NRRL–B–11308 und/oder Sporothrix schenkii NRRL–Y–11307 oder ihren Mutanten die Verbindungen der Formel Ia/Ib total abgebaut werden.

Es werden durch das erfindungsgemässe Verfahren der aeroben Verwendung einer Kombination des Rhodococcus corallinus mit mindestens einem der beiden Pseudomonas spp. zur Behandlung von Abwässern, die s-Triazin-Derivate sowohl der Formel Ia als auch der Formel Ib enthalten, die genannten Substanzen vollständig abgebaut und zur Bildung von Zellmasse verwendet.

Der erfindungsgemässe neue Mikroorganismus Rhodococcus corallinus NRRL B–15444 sowie die bekannten Pseudomonaden spp. NRRL B–12228 und NRRL B–12229 finden Anwendung in erfindungsgemässen Verfahren zur mikrobiologischen Reinigung von wässrigen Lösungen enthaltend Verbindungen der Formeln Ia und/oder Ib, dadurch gekennzeichnet, dass man den Mikroorganismus Rhodococcus corallinus NRRL B–15444 oder eine von diesem Mikroorganismus abgeleitete Mutante, und die Pseudomonaden spp. NRRL B–12228 und NRRL B–12229 oder eine ihrer Mutanten, welche Zellmasse zu erzeugen vermögen, in Gegenwart von wachstums-begünstigenden anorganischen Salzen bei ca. 20° bis ca. 40 °C und einem pH-Wert von ca. 6 bis ca. 8,5 wachsen lässt, und, falls erwünscht, die erhältliche Zellmasse isoliert.

Bei der verfahrensmässigen Behandlung der wässrigen Lösungen bauen die vorstehend genannten Mikroorganismen-Stämme die in diesen Lösungen enthaltenen Verbindungen der Formeln Ia und/oder Ib ab und verbrauchen dabei Sauerstoff, wobei die genannten Bestandteile in den Lösungen auch bis zu Sättigungskonzentrationen vorhanden sein können.

Zur fermentativen Behandlung der Lösungen wird der pH-Wert durch Zugabe von Pufferlösung, beispielsweise Phosphatpuffer, oder von wässriger Base, z. B. wässriger Natrium- oder Kaliumhydroxidlösung, auf Werte zwischen ca. 6 und ca. 8, vorzugsweise 7, eingestellt.

Das Wachstum der Mikroorganismen wird begünstigt durch die Gegenwart gewisser Mengen Salze wie Sulfate oder Phosphate von Alkali-(K, Na), Erdalkali-(Ca, Mg) oder Übergangsmetallen (Fe) sowie Borate oder Molybdate von Alkalimetallen.

Bevorzugte anorganische Salze sind beispielsweise Dinatrium- oder Dikalimhydrogenphosphat, Natrium- oder Kaliumhydrogenphosphat, Magnesium- und Eisensulfat und Kalium- und Calciumchlorid. In geringen Mengen können noch Zink-, Mangan- und Kupfersulfat, Natriummolybdat und Borax zugesetzt werden. Sonstige vorhandene Salze (z. B. Alkalichloride) stören in Konzentrationen bis zu 4% den Abbau nicht.

Zur Reinigung von wässrigen Lösungen, welche Triazin-Derivate der Formeln Ia und/oder Ib enthalten, setzt man wegen ihres geringen Gehaltes an verwertbarem Kohlenstoff den Lösungen eine Kohlenstoffquelle, beispielsweise Glycerin, Essigsäure oder Ethanol, hinzu.

Das Wachstum der Kulturen erfolgt in diesen Medien unter aeroben Bedingungen, beispielsweise unter Sauerstoff- oder Luftzufuhr, und zwar unter Schütteln oder Rühren in Schüttelkolben oder Fermenten. Die Züchtung lässt sich in einem Temperaturbereich von ca. 20 bis ca. 40 °C, vorzugsweise bei ca. 30 bis 35 °C durchführen.

Das Wachstum der Kulturen kann ansatzweise, beispielsweise durch ein- oder mehrmalige Zugabe von Nährlösung, oder kontinuierlich durch laufende Zugabe der Nährlösung erfolgen. Die Grösse der Reaktionsgefässe spielt für den Ablauf der Reaktion keine Rolle, solange die vorstehend genannten Bedingungen eingehalten werden.

Vorzugsweise züchtet man die Kulturen in mehreren Stufen, indem man zunächst eine oder mehrere Vorkulturen z. B. in einem flüssigen Nährmedium herstellt, welche man anschliessend in die eigentliche Hauptkultur überimpft. Eine Vorkultur lässt sich beispielsweise derart herstellen, dass man eine Probe mit Zellmaterial des betreffenden Mikroorganismus, der beispielsweise auf Schräg-Agar aufbewahrt wurde, in eine sterile Nährlösung, die als Substrat s-Triazin-Verbindungen der Formeln Ia und/oder Ib enthält, überträgt und mehrere Tage bei 28° – 30 °C inkubiert. Mit dieser ersten Vorkultur impft man frische Nährlösung an und inkubiert nochmals mehrere Tage bei gleicher Temperatur.

Man kann den Wachstumsverlauf durch Probenentnahme analytisch während der Fermentation verfolgen, z. B. durch Messung der Konzentrationsabnahme der s-Triazin-Verbindungen in der Kulturlösung oder durch Messung des Proteingehalts, welcher ein Mass für das Wachstum des jeweiligen Stamms ist, sowie gravimetrisch anhand des Trockengewichts der gebildeten Zellmasse.

Die gebildete Zellmasse lässt sich anschliessend nach einem der zahlreichen in der Europäischen Patentschrift 10243 beschriebenen Verfahren aufarbeiten und z. B. in hochwertige Düngemittel überführen, die frei sind von kontaminierenden Metallionen.

Als Zellmasse sind im vorliegenden Fall alle sich in lebendem Zustand, z. B. im Teilungszustand bzw. Ruhezustand, im partiellen oder vollständigen Zelltod, oder bereits in der enzymatischen Zersetzung oder in der Zersetzung durch Fremdkulturen befindlichen Zellsysteme zu verstehen, welche von den Mikroorganismen der vorliegenden Anmeldung aufgebaut wurden.

Die verfahrensgemäss erhältliche Zellmasse mit definierter und reproduzierbarer Zusammensetzung kann beispielsweise als Viehfutterzusatz verwendet werden. Sie lässt sich auch, wie erwähnt, als Suspension oder aufgearbeitet, z. B. nach Entwässerung oder Pasteurisierung, als Düngemittel verwenden. Man kann sie auch als Ausgangsmaterial zur Herstellung von Biogas mit hohem Heizwert (Zusammensetzung ca. 70% Methan, 29% Kohlendioxid und 1% Wasserstoff, Heizwert ca. 5500–6500 kcal/$m^3$), beispielsweise durch anaerobe Vergärung in Faultürmen, verwenden. Der Rückstand (Faulschlamm) aus dem Herstellungsverfahren von Biogas ist ebenfalls ein hochwertiger Dünger, der im Vergleich zur ursprünglichen Zellmasse mit Stickstoff stark angereichert ist.

Die folgenden Beispiele illustrieren die vorliegende Erfindung. Die Temperaturangaben erfolgen in Grad Celsius.

Beispiel 1: (Herstellung der Vorkultur)

1 Probe mit auf Schrägagar aufbewahrtem Zellmaterial des Mikroorganismus vom Stamm Rhodococcus corallinus NRRL B–15444 wird in ein Röhrchen, enthaltend 3 ml Nährlösung mit 0,5 mMol 2-Amino-4-ethylamino-6-chlor-s-triazin gegeben und 96 Stunden bei 30° und 4 rps inkubiert. 1 ml dieser ersten Vorkultur wird in einen zweiten Schüttelkolben, enthaltend 100 ml Nährlösung mit 0,5 mMol 2-Amino-4-ethylamino-6-chlortriazin gegeben und 96 Stunden bei 28° und 4 rps inkubiert (rps = Umdrehungen pro Sekunde).

Beispiel 2: Abbau von 2-Amino-4-ethylamino-6-chlor-s-triazin zu 2,6-Dihydroxi-4-ethylamino-s-triazin durch wachsende Kulturen von Rhodococcus corallinus NRRL B–15444

In einen Laborfermenter werden 10 Liter wahlweise hitzesterilisierte (Sterilisation 20 Min. bei 120°) Nährlösung enthaltend 0,5 mMol 2-Amino-4-ethylamino-6-chlor-s-triazin gegeben.

Man gibt eine Probe mit ca. 500 ml der zweiten Vorkultur des Stamms Rhodococcus corallinus NRRL B–15444 hinzu und hält folgende Bedingungen ein: pH-Wert von 7, welcher unter Rühren durch Zugabe jeweils von 4-normaler Natronlauge bzw. 1-normaler Salzsäure gehalten wird, Temperatur: 28 °C, Luftzufuhr 0,26 l/min.

Der Stamm wächst auf reinem 2-Amino-4-ethylamino-6-chlor-s-triazin als einziger Stickstoffquelle. Nach ca. 200 Stunden ist das eingesetzte s-Triazin vollständig abgebaut. Die gebildete Zellmasse liegt als Gemisch von Einzelzellen oder Aggregaten verschiedener Grösse vor, welche man durch Absetzen oder Zentrifugieren abtrennen kann.

Beispiel 3: Abbau von 2-Amino-4-ethylamino-6-chlor-s-triazin zu 2,6-Dihydroxi-4-ethylamino-s-triazin durch wachsende Kulturen von Rhodococcus corallinus NRRL B–15444

Von einer Schrägagar-Kultur auf Basis-Agar wird eine Probe des Stammes Rhodococcus corallinus NRRL B–15444 entnommen und in Basis-Medium folgender Zusammensetzung inoculiert:

| | |
|---|---|
| Kaliumphosphat-Puffer, pH 7,3 | 10 mM |
| Magnesiumsulfat-Heptahydrat | 0,25 mM |
| Spurenelementlösung nach Pfennig und Lippert (Arch. Microbiol. 55, 246, 1966), ergänzt mit 100 mg pro Liter Calciumchlorid-Dihydrat | 5 ml/Liter |
| Glycerin | 10 mM |
| 2-Amino-4-ethylamino-6-chlor-s-triazin | 2,5 mM |

Davon werden Portionen von 60 ml Kulturlösung in je 500 ml Erlenmeyer-Kolben auf einer rotierenden Schüttelmaschine (2–3 rps) bei 30 °C für 75 Stunden inkubiert. In Abständen von 24 Stunden werden Proben entnommen und Messungen der Trübung des Proteingehalts und der Konzentration der Stickstoffquelle durchgeführt.

Ergebnis:

| Protein g/Mol Substrat | Stickstoff Verwertung Mol/Mol Substrat | Protein g/Mol N | Abbauverbindung Mol/ Mol Substrat |
|---|---|---|---|
| 39 | 1 | 39 | 0,98 |

Beispiel 4: Teilweiser Abbau oder Totalabbau (*) verschiedener s-Triazin-Derivate durch wachsende Kulturen von Rh. corallinus NRRL B–15444

Nach Art der in Beispiel 3 angegebenen Versuchsanordnung werden als N-Quellen weitere s-Triazin-Derivate zur Prüfung der Abbauleistungen von Rh. corallinus NRRL B–15444 herangezogen. Die aufgeführten s-Triazin-Derivate werden, wie angegeben, entweder teilweise oder aber vollständig (*), d.h. im wesentlichen zu $CL^-$ und Zellmasse, abgebaut. In diesem zweiten Falle erscheint in der letzten Kolonne der Tabelle «n.n.» (= nicht nachweisbar).

Folgende Abkürzungen für s-Triazin-Derivate werden verwendet:

EOAT = 2-Ethylamino-4-hydroxi-6-amino-s-triazin (wird abgebaut zu EOOT);
IOAT = 2-Isopropylamino-4-hydroxi-6-amino-s-triazin (wird abgebaut zu IOOT);
EOOT = 2-Ethylamino-4,6-dihydroxi-s-triazin;
IOOT = 2-Isopropylamino-4,6-dihydroxi-s-triazin;
AAAT(*) = Melamin (2,4,6-Trisamino-s-triazin);
OAAT(*) = 2-Hydroxi-4,6-diamino-s-triazin (Ammelin);
OOAT(*) = 2,4-Dihydroxi-6-amino-s-triazin (Ammelid);
OOOT(*) = Cyanursäure (2,4,6-Tris-hydroxi-s-triazin).

Tabelle

| N-Quelle | Protein g/Mol Substrat | Stickstoffverwertung Mol/Mol Substrat | Protein g/Mol N | Abbauverbindung Mol/Mol Substrat | N-Quelle (Schluss) |
|---|---|---|---|---|---|
| EOAT | 46 | 1 | 46 | 0,99 | EOOT |
| IOAT | 49 | 1 | 49 | 1,03 | IOOT |
| EOOT | 1 | 0 | 0 | 0 | EOOT |
| IOOT | 1 | 0 | 0 | 0 | IOOT |
| AAAT | 264 | 6 | 44 | n. n. | n. n. |
| OAAT | 246 | 5 | 49 | n. n. | n. n. |
| OOAT | 184 | 4 | 46 | n. n. | n. n. |
| OOOT | 150 | 3 | 50 | n. n. | n. n. |

Wie im folgenden Beispiel 5 gezeigt wird, lässt sich die fehlende Abbauleistung des Rh. corallinus NRRL 5–15444 auf 2-Alkylamino-4,6-dihydroxi-s-triazine der Formel Ic (z. B. EOOT und IOOT), womit der Abbau von 2-Chlor-4-amino-6-alkylamino-s-Triazin-Derivaten auf dieser Stufe zum Stillstand kommt, durch kombinierte Anwendung mit anderen Mikroorganismen, die in der Lage sind, solche s-Triazin-Derivate als N-Quelle zu verwerten, in ein hochleistungsfähiges Totalabbauverfahren für s-Triazin-Derivate der Formel Ia/Ib verwandeln. Dabei werden höhere Abbauleistungen erzielt, als aus den Einzelwerten der separat eingesetzten Mikroorganismen zu erwarten gewesen wäre.

Beispiel 5: Abbau von 2-Amino-4-ethylamino-6-chlor-s-triazin zu Chloridionen und Zellmasse durch gemeinsam wachsende Kulturen von Rhodococcus corallinus NRRL B–15444 und Pseudomonas sp. NRRL B–12228

Von Schrägagarkulturen auf Basis-Agar werden Proben der Stämme Rhodococcus corallinus NRRL B–15444 und Pseudomonas sp. NRRL B–12228 entnommen und gemeinsam in Basis-Medium folgender Zusammensetzung inoculiert:

| | |
|---|---|
| Kaliumphosphat-Puffer, pH 7,3 | 15 mM |
| Magnesiumsulfat-Heptahydrat | 0,25 mM |
| Spurenelementlösung nach Pfennig und Lippert (Arch. Microbiol. 55, 246, 1966), ergänzt mit 100 mg pro Liter Calciumchlorid-Dihydrat | 5 ml/Liter |
| Glycerin | 10 mM |
| Milchsäure | 10 mM |
| 2-Amino-4-ethylamino-6-chlor-s-triazin (= CEAT) | 2,5 mM |

Davon werden Portionen von 60 ml Kulturlösung in je 500 ml Erlenmeyer-Kolben auf einer rotierenden Schüttelmaschine (2–3 rps) bei 30 °C für 75 Stunden inkubiert. In Abständen von 24 Stunden werden Proben entnommen und Messungen der Trübung des Proteingehalts und der Konzentration der Stickstoffquelle durchgeführt.

Ergebnis

| N-Quelle | Protein g/Mol Substrat | Stickstoff-verwertung Mol/Mol Substrat | Protein g/Mol N | Abbau-verbindung Mol/Mol Substrat | N-Quelle (Schluss) |
|---|---|---|---|---|---|
| CEAT | 215 | 5 | 43 | n. n. | n. n. |

**Patentansprüche**

1. Mikroorganismus des Genus Rhodococcus corallinus NRRL B–15444 und seine Mutanten, die dadurch gekennzeichnet sind, dass sie in wässrigen Lösungen aus s-Triazin-Derivaten der Formel Ia und/oder Ib

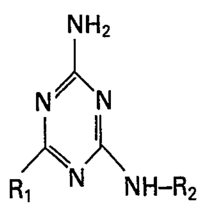

Ia

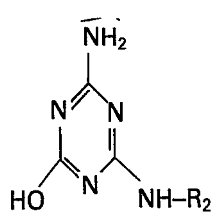

Ib

worin $R_1$ ein Chloratom oder eine $NH_2$-Gruppe und $R_2$ Wasserstoff oder einen aliphatischen oder cycloaliphatischen Kohlenwasserstoffrest mit maximal 4 Kohlenstoffatomen bedeuten, entsprechende Ammelide der Formel Ic

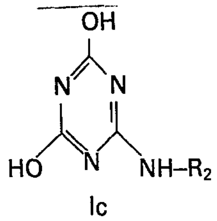

Ic

zu bilden vermögen.

2. Verfahren zum Abbau von s-Triazin-Derivaten der Formeln Ia und Ib

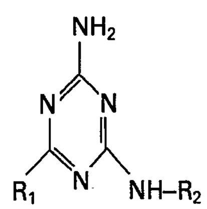

Ia

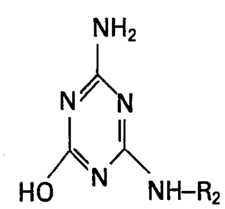

Ib

worin $R_1$ ein Chloratom oder eine $NH_2$-Gruppe und $R_2$ Wasserstoff oder einen aliphatischen oder cycloaliphatischen Kohlenwasserstoffrest mit maximal 4 Kohlenstoffatomen bedeuten, in wässrigen Lösungen, dadurch gekennzeichnet, dass man diese Lösungen unter aeroben Bedingungen im pH-Bereich von 6–8 mit dem Stamm Rhodococcus corallinus NRRL B–15444 in Kontakt bringt.

3. Verfahren gemäss Anspruch 2, wobei in den Formeln Ia und Ib $R_2$ Wasserstoff, Ethyl, Isopropyl oder Cyclopropyl bedeutet.

4. Verfahren gemäss Anspruch 2, wobei in der Formel Ia $R_1$ ein Chloratom bedeutet.

5. Verfahren gemäss Anspruch 2, wobei als für den Mikroorganismus wachstumsbegünstigende anorganische Salze Sulfate oder Phosphate von Alkali-, Erdalkali- oder Übergangs-Metallen oder Alkalimolybdate zugesetzt werden.

6. Verfahren gemäss Anspruch 2, wobei man im Temperaturbereich von 20° bis 45°C arbeitet.

7. Verfahren zur mikrobiologischen Reinigung von wässrigen Lösungen enthaltend Verbindungen der Formeln 1a und/oder 1b

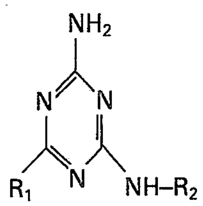

Ia

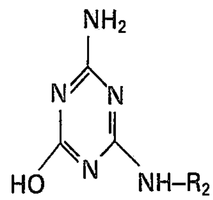

Ib

worin $R_1$ ein Chloratom oder eine $NH_2$-Gruppe und $R_2$ Wasserstoff oder einen aliphatischen oder cycloaliphatischen Kohlenwasserstoffrest mit maximal 4 Kohlenstoffatomen bedeuten, dadurch gekennzeichnet, dass man den Mikroorganismus Rhodococcus coralinus NRRL B-15444 oder eine von diesem Mikroorganismus abgeleitete Mutante, die imstande ist 2,4-Dihydroxy-6-amino-s-triazin-Derivate abzubauen, und die Pseudomonaden spp.NRRL B-12228 und NRRL B-12229 oder eine ihrer Mutanten, die imstande sind 2,4-Dihydroxy-6-amino-s-triazin-Derivate abzubauen, welche Zellmasse zu erzeugen vermögen, in Gegenwart von wachstums-begünstigenden anorganischen Salzen bei ca. 20° bis ca. 40°C und einem pH-Wert von ca. 6 bis ca. 8,5 wachsen lässt, und, falls erwünscht, die erhältliche Zellmasse isoliert.

8. Verfahren gemäss Anspruch 7, dadurch gekennzeichnet, dass unter aeroben Bedingungen im pH-Bereich von 5–7 gearbeitet wird.

9. Verfahren gemäss Anspruch 7, dadurch gekennzeichnet, dass als weitere Mikroorganismen Pseudomonas NRRL-B-11308 und/oder Sporothrix schenkii NRRL-Y-11307 zugesetzt werden.

**Claims**

1. A micro-organism of the genus Rhodococcus corallinus NRRL B-15444 and the mutants thereof which are characterised in that, in aqueous solutions of s-triazine derivatives of the formula Ia and/or Ib

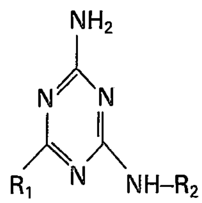

Ia

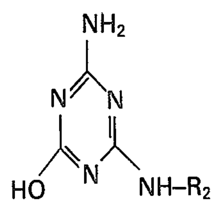

Ib

wherein $R_1$ is a chlorine atom or an $NH_2$ group and $R_2$ is hydrogen or an aliphatic or cycloaliphatic hydrocarbon radical containing not more than 4 carbon atoms, they are able to form corresponding ammelides of the formula Ic

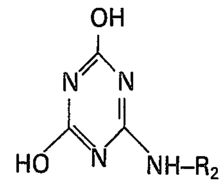

Ic

2. A process for degrading s-triazine derivatives of the formulae Ia and Ib

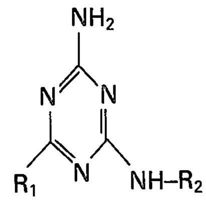

Ia

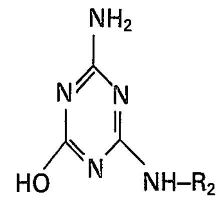

Ib

wherein $R_1$ is a chlorine atom or an $NH_2$ group and $R_2$ is hydrogen or an aliphatic or cycloaliphatic hydrocarbon radical containing not more than 4 carbon atoms, in aqueous solutions, which process is characterised in that these solutions are brought into contact with the strain Rhodococcus corallinus NRRL B-15444, under aerobic conditions, in the pH range of from 6 to 8.

3. A process according to claim 2, wherein $R_1$ is hydrogen, ethyl, isopropyl or cyclopropyl in the formulae Ia and Ib.

4. A process according to claim 2, wherein $R_1$ in formula Ia is a chlorine atom.

5. A process according to claim 2, wherein sulfates or phosphates of alkali metals, alkaline earth metals or transition metals or alkali metal molybdates are added as inorganic salts that promote the growth of the microorganism.

6. A process according to claim 2, wherein the process is carried out in the temperature range of from 20° to 45°C.

7. A process for the microbiological purification of aqueous solutions containing compounds of the formula Ia and/or Ib

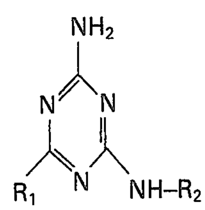

Ia

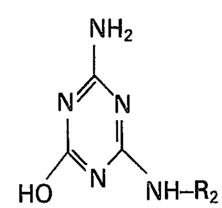

Ib

wherein $R_1$ is a chlorine atom or an $NH_2$ group and $R_2$ is hydrogen or an aliphatic or cycloaliphatic hydrocarbon radical containing not more than 4 carbon atoms, which process is characterised in that the microorganism Rhodococcus corallinus NRRL B-15444, or a mutant derived from that micro-organism, which mutant is able to degrade 2,4-dihydroxy-6-amino-s-triazine derivatives, and Pseudomonas sp. NRRL B-12228 and Pseudomonas sp. NRRL B-12229, or one of the mutants

thereof that are able to degrade 2,4-dihydroxy-6-amino-s-triazine derivatives, which are capable of producing biomass, are grown in the presence of growth-promoting inorganic salts at from about 20° to about 40° C and at a pH value of from about 6 to about 8,5, and, if desired, the resulting biomass is isolated.

8. A process according to claim 7, characterised in that the process is carried out under aerobic conditions in the pH range of from 5 to 7.

9. A process according to claim 7, characterised in that Pseudomonas NRRL B-11308 and/or Sporothrix schenkii NRRL Y-11307 are added as additional micro-organisms.

**Revendications**

1. Microorganisme du genre Rhodococcus corallinus NRRL B-15444 et ses mutants, caractérisés en ce que, dans des solutions aqueuses de dérivés de s-triazines de formules Ia et/ou Ib

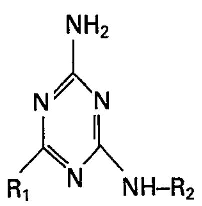

Ia

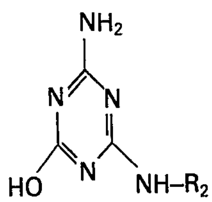

Ib

dans lesquelles $R_1$ représente un atome de chlore ou un groupe $NH_2$ et $R_2$ l'hydrogène ou un radical hydrocarboné aliphatique ou cycloaliqhatique contenant au maximum 4 atomes de carbone, ils sont capables de former les ammélides correspondants de formule Ic

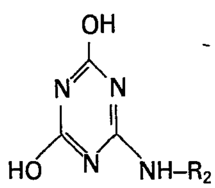

Ic

2. Procédé pour la dégradation des dérivés de s-triazines de formules Ia et Ib

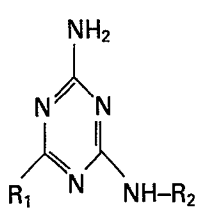

Ia

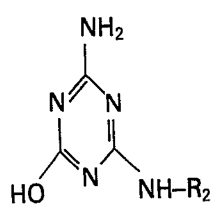

Ib

dans lesquelles $R_1$ représente un atome de chlore ou un groupe $NH_2$ et $R_2$ l'hydrogène ou un radical hydrocarboné aliphatique ou cycloaliphatique contenant au maximum 4 atomes de carbone, dans des solutions aqueuses, caractérisé en ce que l'on met ces solutions en contact dans des conditions aérobies et dans un intervalle de pH de 6 à 8 avec la souche Rhodococcus corallinus NRRL B-15444.

3. Procédé selon la revendication 2, dans lequel le symbole $R_2$ des formules Ia et Ib représente l'hydrogène, un groupe éthyle, isopropyle ou cyclopropyle.

4. Procédé selon la revendication 2, dans lequel le symbole $R_1$ de la formule Ia représente un atome de chlore.

5. Procédé selon la revendication 2, dans lequel on ajoute en tant que sels minéraux favorisant la croissance du microorganisme des sulfates ou phosphates de métaux alcalins, alcalino-terreux ou de transition ou des molybdates alcalins.

6. Procédé selon la revendication 2, dans lequel on opère dans un intervalle de température de 20 à 45°C.

7. Procédé pour l'épuration microbiologique de solutions aqueuses contenant des composés de formules Ia et/ou Ib

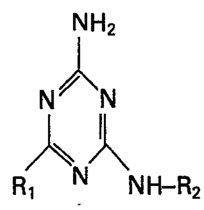

Ia

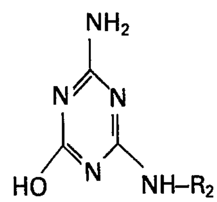

Ib

dans lesquelles $R_1$ représente un atome de chlore ou un groupe $NH_2$ et $R_2$ l'hydrogène ou un radical hydrocarboné aliphatique ou cycloaliphatique contenant au maximum 4 atomes de carbone, caractérisé en ce que l'on fait croître le microorganisme Rhodococcus corallinus NRRL B-15444 ou l'un des mutants dérivés de ce microorganisme et capables de dégrader les dérivés de 2,4-dihydroxy-6-amino-s-triazines, et les Pseudomonas spp. NRRL B-12228 et NRRL B-12229 ou l'un de leurs mutants capables de dégrader les dérivés de 2,4-dihydroxy-6-amino-s-triazines, et capables de produire une masse cellulaire, en présence de sels minéraux favorisant la croissance, à des températures d'environ 20 à 40°C et à un pH d'environ 6 à 8,5 et si on le désire, on isole la masse cellulaire obtenue.

8. Procédé selon la revendication 7, caractérisé en ce que l'on opère dans des conditions aérobies dans un intervalle de pH de 5 à 7.

9. Procédé selon la revendication 7, caractérisé en ce que l'on ajoute en tant qu'autres microorganismes Pseudomonas NRRL B-11308 et/ou Sporothrix schenkii NRRL-Y-11307.